# FASCICULE DE BREVET EUROPEEN

(11) **EP 1 851 326 B1**
(45) Date de publication et mention de la délivrance du brevet: **09.12.2009**
(21) Numéro de dépôt: 06708421.0
(22) Date de dépôt: 21.02.2006
(51) Int. Cl.: C12Q 1/04, C12Q 1/10

(54) **DETECTION D'UNE SOUCHE DE MICROORGANISMES DANS UN ECHANTILLON LIQUIDE**
NACHWEIS EINES MIKROORGANISMUSSTAMMS IN EINER FLÜSSIGKEITSPROBE
DETECTING A MICROORGANISM STRAIN IN A LIQUID SAMPLE

(30) Priorité: 22.02.2005 FR 0501764
(43) Date de publication de la demande: 07.11.2007
(73) Titulaire: Rambach, Alain, F-75006 Paris (FR)
(72) Inventeur: Rambach, Alain, F-75006 Paris (FR)
(74) Mandataire: Ahner, Francis
(86) Numéro de dépôt international: PCT/EP2006/060143
(87) Numéro de publication internationale: WO 2006/089889

(56) Documents cités:
- WO-A-96/38584
- WO-A-96/40861
- WO-A-99/50438
- WO-A-03/050289
- WO-A-2004/027086
- DE-A1- 4 324 392
- US-A- 5 411 867
- US-A- 5 434 056
- US-A- 5 726 031
- US-A- 5 780 259
- US-A- 5 861 270
- US-A- 5 935 799
- US-A- 5 962 251
- US-A1- 2002 090 668

## Description

La présente invention a pour objet un procédé de détection, d'identification et de différenciation d'une souche de microorganismes dans un échantillon liquide.

La détection de microorganismes pathogènes et indicateurs divers, en particulier de l'eau, préoccupe les microbiologistes depuis de nombreuses années.

Ceux-ci se sont en effet attachés à développer des techniques pour détecter non seulement des indicateurs de contamination fécale tels que *E. coli,* les coliformes, *Enterococcus* mais également des pathogènes tels que *Aeromonas.*

Les bactéries *E. coli* font partie du groupe des coliformes. C'est une espèce très abondante dans la flore intestinale humaine et animale et il est admis que cette espèce est la seule qui soit strictement d'origine fécale. Les bactéries *E*. *coli* sont considérées comme le meilleur indicateur de contamination fécale et leur présence dans l'eau signifie que cette dernière est contaminée par une pollution d'origine fécale et qu'elle est donc susceptible de contenir d'autres microorganismes pathogènes. La gastroentérite est la maladie la plus fréquente associée à l'ingestion d'eau contaminée par les matières fécales. Bien que cette maladie soit souvent bénigne, elle peut parfois avoir des conséquences très graves sur la santé. D'autres maladies plus rares comme les hépatites ou les méningites peuvent aussi être provoquées par l'ingestion d'eau contaminée.

Avant l'invention des milieux chromogènes, la détection de *E. coli* et des autres coliformes se faisait par l'étude complexe d'une multiplicité de caractères tels que la fermentation du lactose, la production d'acide et de gaz....

Les coliformes appartiennent en fait à la famille des *Enterobacteriaceae,* Gram non sporulantes et il est communément admis qu'ils englobent différents genres, tels que *Enterobacter, Klebsiella. Citrobacter* et *Escherichia.*

Il a ensuite été démontré que tous les microorganismes appartenant à ce groupe possédaient une activité β-galactosidase, les *E. coli* typiques possédant en plus une activité β-glucuronidase.

La fin des années 70 a connu l'émergence timide de galeries d'identification de microorganismes utilisant des substrats chromogènes, technique basée sur le fait que chaque souche de microorganisme possède une ou plusieurs activité(s) enzymatique(s) (telles que la β-glucuronidase, la β-galactosidase, l'α-galactosidase, la β-glucosaminidase, les estérases, les phosphatases, etc...) susceptible(s) d'agir sur un chromogène, lequel libère alors un chromophore donnant lieu à une coloration.

Les années 90 ont ensuite connu le développement des milieux d'isolement de microorganismes utilisant des substrats chromogènes précipitant.

Compte tenu que les normes de qualité des eaux de baignade sont données pour 100 ml d'eau, on a coutume de tester un échantillon de 100 ml d'eau pour détecter la présence/absence ou effectuer un dénombrement de microorganismes. Cependant, il est à noter que les normes en vigueur relativement à l'eau potable imposent une qualité microbiologique telle, parmi plus de 60 autres critères, que l'eau ne doit contenir ni parasite, ni virus, ni bactérie pathogène et doit être exempte de *E. coli* dans 100 ml.

Par conséquent, des contrôles doivent constamment être effectués sur l'ensemble du réseau de distribution de l'eau, à savoir les points de captage, les stations de traitement, les réservoirs et les réseaux afin de prévenir un quelconque risque de contamination de l'eau d'origine animale ou humaine.

Certaines méthodes de détection de microorganismes contaminant l'eau reposent sur la filtration d'un échantillon de 100 ml sur des membranes filtrantes laissant passer l'eau mais retenant les microorganismes. Ces membranes sont ultérieurement transportées sur des milieux de culture solides gélifiés par un gel d'Agar-Agar ou d'autres gélifiants ou dans des tampons solides tels que du papier (technique des filtres absorbants) ou un autre composant spongieux.

Dans ces techniques, les différentes souches présentes dans l'échantillon testé sont isolées les unes des autres, se développent sous forme de colonies bactériennes sur la surface dudit filtre et sont ensuite comptées et identifiées.

Ces méthodes sont largement utilisées et donnent des résultats satisfaisants quand elles sont combinées à des réactifs spécifiques. Elles présentent cependant l'inconvénient d'être coûteuses et d'être longues à mettre en oeuvre.

Une autre méthode utilise des milieux non gélifiés et repose sur le test direct de l'échantillon liquide à tester auquel on ajoute le milieu (c'est le cas par exemple du test Colilert® commercialisé par la Société IDEXX ou du test Readycult® commercialisé par la Société MERCK). Elle est réalisée dans un récipient unique afin d'obtenir un résultat qualitatif (présence/absence) ou dans des tubes ou compartiments multiples pour obtenir un résultat quantitatif comme c'est le cas pour la méthode MPN (Most Probable Number) utilisée pour estimer le nombre de coliforms et de *E*. *coli* nécessitant toutefois un matériel spécifique ainsi que du temps de manipulation.

De plus, cette méthode présente l'inconvénient, au moins dans le cadre de la détection de *E*. *coli* et des coliformes, de nécessiter un substrat fluorogène.

En effet, les techniques qui associent un substrat de la β-galactosidase pour détecter les coliformes et un substrat de la glucuronidase pour détecter *E*. *coli* mettent généralement en oeuvre un substrat enzymatique chromogène pour détecter les coliformes et un substrat enzymatique fluorogène pour détecter *E*. *coli.* La détection de *E*. *coli* nécessite dans ce cas d'effectuer une lecture de l'échantillon dans des conditions particulières dans la mesure où elle requiert que la fluorescence soit détectée en chambre noire sous une lumière UV.

De plus, cette association « chromogène + fluorogène » ne permet de différencier, au moyen d'une couleur et de la fluorescence, que deux types de microorganismes définis par des capacités enzymatiques respectives qui permettent clairement de les différencier l'un de l'autre.

Il doit également être noté que les techniques de l'art antérieur utilisant des produits non gélifiants, dans lesquels les colonies ne sont pas isolées comme elles le sont sur des milieux gélifiés, ne permettent pas de différencier par exemple le pathogène *Aeromonas* qui est, à l'instar des coliformes, un microorganisme positif pour la β-galactosidase. Ainsi, il est généralement proposé d'ajouter de la cefsulodine ou un autre agent antimicrobien sélectif dans l'échantillon à analyser, au risque de ne pas détecter du tout *Aeromonas* et au risque d'inhiber au moins partiellement certains *E. coli.*

De plus, les combinaisons « chromogène + fluorogène » de l'art antérieur ne permettent pas de repérer les *E. coli* glc⁻ (*E. coli* atypiques négatives pour la glucuronidase) qui représentent environ 5 % des *E*. *coli.*

Par exemple le brevet US 5,935,799 décrit l'utilisation d'un agent chromogénique pour identifier les coliformes (colonne 4, dernier paragraphe) ou l'utilisation d'un agent flurogénique pour détecter E.coli. La méthode de détection décrite dans le document US 5,935,799 ne permet pas une détection unique du fluorogène et du chromogène, mais plutôt deux étapes de détection qui s'opèrent chacune à des longueurs d'onde bien distinctes.

La présente invention propose de remédier aux inconvénients de l'art antérieur par l'utilisation d'une combinaison de chromogènes substrats d'enzymes capables de libérer des chromophores sous l'effet de ces enzymes, ladite combinaison étant choisie pour permettre la détection, l'identification et la différenciation d'une souche de microorganismes dans un échantillon liquide. Bien évidemment, la combinaison desdits chromogènes est à déterminer en fonction des différentes souches de microorganismes que l'on souhaite détecter et plus particulièrement, des activités enzymatiques respectives de ladite souche.

La présente invention a en effet pour objet un procédé de détection, d'identification et de différenciation d'au moins une souche de microorganismes choisie dans le groupe comprenant les souches : *E. coli glc⁻,* les souches *E. coli* typiques, les coliformes autres que *E. coli* ou autres que *E. coli* typiques et les bactéries du genre *Aeromonas,* dans un échantillon liquide susceptible de contenir au moins l'une de ces souches, ledit procédé comprenant:
a) le mélange de l'échantillon liquide avec un milieu de sorte d'obtenir un mélange liquide comprenant:
   - les nutriments nécessaires à l'incubation de ladite au moins une souche à détecter,
   - au moins deux chromogènes, chacun desdits chromogènes étant soit le substrat d'une enzyme exprimée par ladite au moins une souche à détecter soit le substrat d'une enzyme exprimée par une autre souche susceptible de contaminer ledit échantillon et libérant chacun un chromophore sous l'effet de cette enzyme, lesdits chromophores contribuant à la couleur finale dudit mélange liquide,
b) l'incubation du mélange obtenu a l'étape a) pendant 18 à 24 heures à une température de 34°C à 40°C et de préférence de 37°C,
c) la soumission du mélange incubé au rayonnement de la lumière et la lecture de la couleur finale dudit mélange dans les longueurs d'onde du visible, et
d) l'identification de ladite au moins une souche de microorganismes en fonction de ladite couleur finale.

Par « souche » ou « souche de microorganismes », on entend indifféremment une espèce particulière ou encore un groupe de microorganismes connus pour avoir des propriétés communes et couramment identifiés par un terme commun.

Ainsi, dans le cadre de la présente invention, l'expression « souche » ou « souche de microorganismes », s'applique notamment aux souches *E. coli* glc⁻, les souches *E. coli* typiques (c'est-à-dire *E. coli* glc⁺), les coliforms autres que *E. coli* ou autres que *E. coli* typiques et les bactéries du genre *Aeromonas.* Cette expression concerne également les groupes de souches de microorganismes parmi celles citées précédemment telles que par exemple « *E. coli* typiques + autres coliformes » ou « *E. coli* + autres coliformes ».

Par « nutriments nécessaires à l'incubation de la souche à détecter », on entend la composition d'un milieu de base nécessaire à la croissance de ladite souche. L'homme du métier connaît parfaitement la composition de tels milieux et est à même de l'adapter si nécessaire en fonction de la spécificité de certaines souches. Ces nutriments sont notamment choisis dans le groupe comprenant du carbone, de l'azote, du souffre, du phosphore, des vitamines, des inducteurs de croissance, des hydrates de carbone, des sels (par exemple calcium, magnésium, manganèse, sodium, potassium), des complexes nutritifs (par exemple des acides aminés, du sang, du sérum, de l'albumine) ainsi que des peptones et des extraits de tissus animaux et végétaux.

Il est à souligner que la détection, l'identification et la différenciation d'une souche de microorganismes, dans le cadre de la présente invention, est réalisée dans un mélange non gélifié (constitué de l'échantillon liquide et du milieu décrit précedemment) dans lequel les microorganismes ne sont pas séparés les uns des autres, comme le sont des colonies isolées sur un milieu gélifié. De plus, la présente invention ne nécessite pas l'ajout de substrats fluorogènes pour différencier une souche de microorganismes d'une autre et la coloration finale obtenue (après une période d'incubation) est visible dans les longueurs d'ondes du visible.

En fait, après incubation, le mélange constitué du milieu de décrit précédemment et de l'échantillon liquide est soumis au rayonnement de la lumière, c'est-à-dire qu'il est placé dans un lieu où il peut être atteint par les longueurs d'ondes du visible et la coloration finale de ce mélange est détectable également dans les longueurs d'ondes du visible, c'est-à-dire à l'oeil nu. On admet que le domaine du spectre visible s'étend environ de 400 à 800 nm.

Par conséquent, la lecture du test est immédiate et simplifiée sans la contrainte de deux lectures successives. De plus, elle ne nécessite aucun dispositif particulier, telle qu'une source de lumière UV. Ainsi le milieu et les matériaux du récipient au moyen desquels seront réalisés la détection, et même le contenu de l'échantillon, peuvent indifféremment générer une fluorescence ou interférer avec elle par effet de quenching, ceci ne gênera en aucune manière la lecture du test.

Dans le cadre de la présente invention, il est à noter que les chromogènes utilisées ne sont pas nécessaires à la croissance des souches à détecter. En effet, pendant la période d'incubation, les souches se développent sur des nutriments traditionnels bien connus de l'homme du métier. De plus, les chromogènes utilisées dans le cadre de la présente invention sont indifféremment non précipitants, précipitants sans ajout ou précipitants après réaction avec un sel du milieu.

Le milieu décrit précédemment peut se présenter sous forme solide ou liquide, déjà introduit dans le récipient où le test sera réalisé ou conditionné dans un récipient indépendant, prêt à être mélangé à l'échantillon liquide à tester.

Dans le cadre du susdit procédé, la mise en contact de l'échantillon liquide avec le milieu décrit précédemment se fait soit par l'ajout du milieu à l'échantillon liquide, soit par l'ajout de l'échantillon liquide au milieu préalablement introduit dans le récipient dans lequel le test sera réalisé.

Ensuite, l'étape de détection d'une souche de microorganismes est précédée de l'incubation du mélange constitué par l'échantillon liquide et le milieu décrit précedemment. Cette étape d'incubation peut être réalisée à une température d'environ 34°C à 40°C et de préférence 37°C et pendant une durée d'environ 18 à 24 h. Cependant, en fonction des moyens dont il disposera, l'homme du métier adaptera la durée de cette étape d'incubation à la température à laquelle il pourra procéder à cette incubation.

Pour ce qui concerne l'étape c) du procédé de l'invention, il n'y aura la plupart du temps aucune démarche particulière à entreprendre pour sa mise en oeuvre dès lors que le test est réalisé par exemple en plein jour à l'extérieur ou à l'intérieur dans une pièce comprenant une entrée de lumière.

Il est à noter que le procédé de l'invention, tout en pouvant être mis en oeuvre de façon totalement manuelle, peut également être mis en oeuvre de façon semi-automatisée voire totalement automatisée.

Dans le cadre de la présente invention, l'échantillon liquide ou liquéfié dans lequel sont réalisées la détection, l'identification et la différenciation d'une souche de microorganismes, est de préférence l'eau et plus préférentiellement l'eau potable. Cependant, cette détection peut également être réalisée dans d'autres liquides et notamment alimentaires tels que le lait, les jus de fruits ou toute autre boisson.

La présente invention permet ainsi de détecter et de différencier non seulement les *E. coli* typiques mais aussi les *E. coli* glucuronidase négatives (glc⁻) sans avoir à soumettre tous les négatifs pour le caractère glc à un test supplémentaire d'indole qui donne lieu à des erreurs pour certains coliformes tels que *Klebsiella oxytoca* comme s'ils étaient des *E. coli* glc⁻. Ce test d'indole est d'ailleurs souvent difficile voire impossible à mettre en oeuvre comme c'est le cas dans le système du Quanti-Tray® (commercialisé par la Société IDEXX) où l'échantillon est placé dans des compartiments fermés et soudés.

La présente invention permet également de distinguer *E. coli* des coliformes autres que *E*. *coli* sans les confondre avec *Aeromonas* et sans avoir besoin d'ajouter de la cefsulodine ou un autre agent antimicrobien inhibant non seulement les *Aeromonas* mais également partiellement les *E*. *coli.*

La présente invention permet en effet de détecter et de différencier simultanément non seulement des indicateurs de contamination fécale tel que *E*. *coli* et les coliformes autres que *E. coli* mais également le pathogène *Aeromonas.*

Le test de détection proposé par la présente invention est essentiellement un test qualitatif, c'est-à-dire un test permettant de révéler la présence ou l'absence d'une souche de microorganismes dans un échantillon liquide. Toutefois, rien ne s'oppose à ce que le test de l'invention soit modifié en un test quantitatif conformément à la méthode MPN par exemple.

Dans le cadre de la présente invention, il convient de déterminer la combinaison de chromogènes appropriée pour la détection de la souche que l'on souhaite détecter. Ainsi, on choisira par exemple un chromogène qui libérera un chromophore de couleur jaune sous l'effet d'une enzyme exprimée par les coliformes autres que *E. coli* et un chromogène libérant un chromophore de couleur bleue sous l'effet d'une enzyme exprimée par les *E. coli.* Si ensuite l'échantillon liquide dans lequel le test est réalisé révèle une coloration finale bleue, on en déduira que l'échantillon est contaminé par des *E. coli* et si la coloration finale est jaune, on en déduira que l'échantillon est contaminé par des coliformes autres que *E. coli.* Il est également à souligner que dans le cas où l'échantillon serait contaminé à la fois par des *E. coli* et des coliformes autres que *E. coli,* la coloration finale obtenue sera dans la gamme des verts.

Il convient donc de choisir les chromophores en fonction de la couleur que l'on souhaite retrouver dans le cas d'une contamination par l'une ou l'autre des souches de microorganismes à détecter.

Le choix de la combinaison des chromogènes est primordial mais il n'est nullement nécessaire que les enzymes agissant sur ces chromogènes soient spécifiques d'une souche de microorganismes. Dans certains cas, on utilisera le caractère négatif pour certaines enzymes de la souche à détecter pour que la couleur finale soit représentative de ladite souche, selon le ou les chromophore(s) libéré(s).

Au cas où l'échantillon liquide ou liquéfié testé contiendrait une souche de microorganismes n'ayant pas d'enzyme correspondant aux substrats présents dans le milieu décrit précedemment et par conséquent ne donnant pas lieu à la libération d'un chromophore, la présence de ladite souche pourrait toutefois être détectée par comparaison avec un échantillon liquide témoin non contaminé. En effet, l'échantillon contaminé serait « trouble » et aurait un aspect laiteux significatif de la croissance de microorganismes dans ledit échantillon.

Bien évidemment, conformément à la présente invention, il est possible d'envisager un grand nombre de combinaisons non seulement pour ce qui concerne les enzymes dont l'activité est utilisée à l'encontre des chromogènes sélectionnés mais également pour ce qui concerne lesdits chromogènes.

Par exemple, parmi les enzymes dont l'activité est utilisable dans le cadre de la présente invention, on peut citer notamment : la β-D-galactosaminidase, la β-D-glucosaminidase, la β-D-cellobiosidase, la β-D-fucosidase, la α-L-fucosidase, la α-D-galactosidase, la β-D-galactosidase, la β-D-lactosidase, la α-D-maltosidase, la α-D-mannosidase, la α-D-glucosidase, la β-D-glucosidase, la β-D-xylosidase, l'estérase, l'acétate estérase, la butyrate estérase, la carboxylestérase, la caprylate estérase, la choline estérase, la myo-inositol phosphatase, la palmitate estérase, la phosphatase, la diphosphatase, l'aminopeptidase et la sulfatase.

Pour ce qui concerne les chromophores dont on souhaite obtenir la libération par la mise en oeuvre d'une activité enzymatique d'une ou plusieurs souche(s) de microorganismes à détecter, on peut citer: O-nitrophényl, P-nitrophényl, ChloroNitrophényl, Hydroxyphényl, Nitroanilide, Phénolphtaléine et thymophtaléine, Hydroxyquinoline, Cyclohexenoesculétine, Dihydroxyflavone, Catéchol, Résazurin, résofurin, VBzTM, VLM, VLPr, VQM, Indoxyl, 5-bromo-4-chloro-3-indoxyl, 5-bromo-6-chloro-3-indoxyl, 6-chloro-3-indoxyl, 6-fluoro-3-indoxyl, 5-Iodo-3-indoxyl, N-Méthylindoxyl.

Comme précédemment indiqué, la présente invention permet de détecter, identifier et différencier la souche *E. coli* dans un échantillon liquide, y compris quand une autre souche est également présente dans ledit échantillon. L'inventeur a toutefois fait l'observation surprenante que, même mélangée à un million de fois plus de coliformes *Enterobacter,* la souche *E. coli* pouvait être détectée après environ 24 heures d'incubation. La combinaison des chromogènes était la suivante : 5-bromo-4-chloro-3-indoxyl glucuronide, substrat de la β-glucuronidase et le nitrophényl β-galactoside substrat de la β-galactosidase. La couleur bleu-vert a indiqué la présence de la souche *E. coli* parmi les coliformes *Enterobacter* (rapport 1 : 1 000 000 entre les deux souches).

Les exemples qui suivent illustrent la présente invention.

### EXEMPLES :

Les exemples ci-après ne représentent que quelques combinaisons de chromogènes choisis pour être des substrats d'enzymes des souches à détecter mais toutes les autres combinaisons découlant directement ou indirectement de l'enseignement de la présente description font également partie de la présente invention.

Pour tous les exemples suivants, le test est réalisé avec 100 ml d'eau et l'étape d'incubation des souches de microorganismes à détecter a été réalisée avec un milieu comprenant au titre des nutriments :
(en gramme(s) par litre) : peptone 5, pyruvate 1, NaCl 5, K₂HPO₄ 4, KH₂PO₄ 1, SDS 0,1, KNO₃ 0,005, Tryptophane 1, Vancomycine 0,002.

Dans le cas de l'exemple 15, le susdit milieu ne contient ni SDS, ni vancomycine.

Pour tous les exemples suivants : l'incubation a été réalisée à 35-37°C pendant environ 24 h.

### Exemple 1 :

La combinaison des chromogènes est la suivante :

| **CHROMOGENES :** | **SUBSTRATS DES ENZYMES :** |
|---|---|
| 5-bromo-4-chloro-3-indoxyl glucuronide (XGlc) | β-glucuronidase |
| + | |
| p-nitrophényl-α-galactoside (pNPaGal) | α-galactosidase |

| **ACTIVITES ENZYMATIQUES : Glc /** α**Gal** | |
|---|---|
| **Souche de microorganismes présente dans l'échantillon liquide** | **Combinaison de chromogènes : Xglc + pNP** α**Gal** |
| *E. coli* | Vert |
| *E. coli* + autres coliformes | Vert |
| Coliformes autres que *E*. *coli* | Jaune |

### Exemple 2 :

La combinaison des chromogènes est la suivante :

| **CHROMOGENES: SUBSTRATS DES ENZYMES:** | |
|---|---|
| 5-bromo-4-chloro-3-indoxyl glucuronide (XGlc) + | β-glucuronidase |
| p-nitrophényl α-galactoside (pNPαGal) + | α-galactosidase |
| 5-bromo-6-chloro-3 indoxyl β-glucoside (Mag βGlu) | β-glucosidase |

| **ACTIVITES ENZYMATIQUES : Glc /** α**Gal /** β**Glu** | |
|---|---|
| **Souche de microorganismes** | **Combinaison de chromogènes :** |
| **présente dans l'échantillon liquide** | **Xglc + pNP** α**Gal + Mag** β**Glu** |
| *E. coli* glc⁻ | Jaune |
| *E. coli* typiques | Vert |
| *E. coli* typiques + autres coliformes | Bleu |
| Coliformes autres que *E. coli* | Orange |
| *Aeromonas* | Mauve |

### Exemple 3 :

Les mêmes enzymes que dans l'exemple 2 ont été utilisées mais deux chromophores ont été inversés.

La combinaison des chromogènes est la suivante :

| **CHROMOGENES :** | **SUBSTRATS DES ENZYMES :** |
|---|---|
| 5-bromo-4-chloro-3-indoxyl glucuronide (XGlc) + | β-glucuronidase |
| 5-bromo-6-chloro-3 indoxyl α-galactoside (Mag αGal) + | α-galactosidase |
| p-nitrophényl β-glucoside (pNPβGlu) | β-glucosidase |

| **ACTIVITES ENZYMATIQUES : Glc /** α**Gal /** β**Glu** | |
|---|---|
| **Souche de microorganismes présente** **dans l'échantillon liquide** | **Combinaison de chromogènes :** **Xglc + Mag** α**Gal + pNP** β**Glu** |
| *E* .*coli* glc⁻ | Mauve |
| *E. coli* typiques | Bleu |
| *E. coli* typiques + autres coliformes | Bleu foncé |
| Coliformes autres que *E*. *coli* | Orange |
| *Aeromonas* | Jaune |

### Exemple 4 :

La combinaison des chromogènes est la même que celle de l'exemple 2 mais il a été ajouté, au milieu décrit précédemment, un inhibiteur du pathogène *Aeromonas* pouvant être indifféremment choisi parmi la cefsulodine 0,005 g/l et l'acide nalidixique 0,001 g/l.

| **ACTIVITES ENZYMATIQUES: Glc /** α**Gal /** β**Glu / inhibiteur de *Aeromonas*** | |
|---|---|
| **Souche de microorganismes présente dans l'échantillon liquide** | **Combinaison de chromogènes : Xglc + pNP** α**Gal + Mag** β**Gglu** |
| *E. coli* glc⁻ | Jaune |
| *E. coli* typiques | Vert |
| *E. coli* typiques + autres coliformes | Bleu |
| Coliformes autres que *E. coli* | Orange |

### Exemple 5 :

La combinaison des chromogènes est la suivante :

| **CHROMOGENES :** | **SUBSTRATS DES ENZYMES :** |
|---|---|
| 5-bromo-4-chloro-3-indoxyl glucuronide (XGlc) + | β-glucuronidase |
| p-nitrophényl α-galactoside (pNPαGal) + | α-galactosidase |
| 5-bromo-6-chlom-3 indoxyl β-galactoside (Mag βGal) | β-galactosidase |

| **ACTIVITES ENZYMATIQUES: Glc /** α**Gal /** β**Gal** | |
|---|---|
| **Souche de microorganismes** | **Combinaison de chromogènes :** |
| **présente dans l'échantillon liquide** | **Xglc + pNP** α**Gal + Mag** β**Gal** |
| *E. coli* | Bleu foncé |
| *E. coli* + autres coliformes | Bleu foncé |
| Coliformes autres que *E. coli* | Orange |
| *Aeromonas* | Mauve |

### Exemple 6 :

Les mêmes enzymes que celles de l'exemple 5 ont été utilisées mais deux chromophores ont été inversés. La combinaison des chromogènes est la suivante :

| **CHROMOGENES:** | **SUBSTRATS DES ENZYMES:** |
|---|---|
| 5-bromo-4-chloro-3-indoxyl glucuronide (XGlc) + | β-glucuronidase |
| 5-bromo-6-chloro-3 indoxyl α-galactoside (Mag αGal) + | α-galactosidase |
| p-nitrophényl β-galactoside (pNPβGal) | β-galactosidase |

| **ACTIVITES ENZYMATIQUES : Glc /** α**Gal /** β**Gal** | |
|---|---|
| **Souche de microorganismes** **présente dans l'échantillon liquide** | **Combinaison de chromogènes :** **Xglc + Mag** α**Gal + pNP**β**Gal** |
| *E. coli* | Bleu foncé |
| *E. coli* + autres coliformes | Bleu foncé |
| Coliformes autres que *E*. *coli* | Orange |
| *Aeromonas* | Jaune |

### Exemple 7 :

La combinaison des chromogènes est la même que celle de l'exemple 5 mais il a été ajouté, au milieu décrit précedemment, un inhibiteur du pathogène *Aeromonas* pouvant être indifféremment choisi parmi la cefsulodine 0,005 g/l et l'acide nalidixique 0,001 g/l.

| **ACTIVITES ENZYMATIQUES: Glc /** α**Gal** β**Gal / inhibiteur de *Aeromonas*** | |
|---|---|
| **Souche de microorganismes présente dans l'échantillon liquide** | **Combinaison de chromogènes : Xglc + pNP** α**Gal + Mag** β**Gal** |
| *E. coli* | Bleu foncé |
| *E. coli* + autres coliformes | Bleu foncé |
| Coliformes autres que *E*. *coli* | Orange |

### Exemple 8 :

La combinaison des chromogènes est la suivante :

| **CHROMOGENES :** | **SUBSTRATS DES ENZYMES :** |
|---|---|
| 5-bromo-4-chloro-3-indoxyl glucuronide (XGlc) + | β-glucuronidase |
| p-nitrophényl β-galactoside (pNPβGal) | β-galactosidase |

| **ACTIVITES ENZYMATIQUES: Glc /** β**Gal** | |
|---|---|
| **Souche de microorganismes présente dans l'échantillon liquide** | **Combinaison de chromogènes : Xglc + pNP** β**Gal** |
| *E. coli* | Bleu vert |
| *E. coli* + autres coliforms | Bleu vert |
| Coliformes autres que *E*. *coli* | Jaune |

### Exemple 9 :

Les enzymes sont les mêmes que dans l'exemple 8 mais un chromophore est différent. La combinaison des chromogènes est la suivante :

| **CHROMOGENES :** | **SUBSTRATS DES ENZYMES :** |
|---|---|
| 5-bromo-4-chloro-3-indoxyl glucuronide (XGlc) + | β-glucuronidase |
| 5-bromo-6-chloro-3-indoxyl β-gatactoside (Mag βGal) | β-galactosidase |

| **ACTIVITES ENZYMATIQUES : Glc /** β**Gal** | |
|---|---|
| **Souche de microorganismes présente dans l'échantillon liquide** | **Combinaison de chromogènes : Xglc + Mag** β**Gal** |
| *E. coli* | Bleu foncé |
| *E. coli* + autres coliformes | Bleu foncé |
| Coliformes autres que *E*. *coli* | Mauve |

### Exemple 10 :

La combinaison des chromogènes est la suivante :

| **CHROMOGENES :** | **SUBSTRATS DES ENZYMES :** |
|---|---|
| 5-bromo-4-chloro-3-indoxyl glucuronide (XGlc) + | β-glucuronidase |
| p-nitrophényl β-galactoside (pNPβGal) + | β-galactosidase |
| 5-bromo-6-chloro-3-indoxyl β-glucoside (Mag βGlu) | β-glucosidase |

| **ACTIVITES ENZYMATIQUES: Glc /** β**Gal /** β**Glu** | |
|---|---|
| **Souche de microorganismes** | **Combinaison de chromogènes :** |
| **présente dans l'échantillon liquide** | **Xglc + pNP** β**Gal + Mag** β**Glu** |
| *E. coli* glc⁻ | Jaune |
| *E. coli* typiques | Vert |
| *E. coli* typiques + autres coliformes | Bleu |
| Coliformes autres que *E. coli* | Orange |
| *Aeromonas* | Mauve |

### Exemple 11 :

Les enzymes sont les mêmes que dans l'exemple 10 mais deux chromophores ont été inversés. La combinaison des chromogènes est la suivante :

| **CHROMOGENES :** | **SUBSTRATS DES ENZYMES :** |
|---|---|
| 5-bromo-4-chloro-3-indoxyl glucuronide (XGlc) + | β-glucuronidase |
| 5-bromo-6-chloro-3-indoxyl β-galactoside (Mag βGal) + | β-galactosidase |
| p-nitrophényl β-glucoside (pNPβGlu) | β-glucosidase |

| **ACTIVITES ENZYMATIQUES : Glc /** β**Gal /** β**Glu** | |
|---|---|
| **Souche de microorganismes présente dans l'échantillon liquide** | **Combinaison de chromogènes : Xglc + Mag** β**Gal + pNP** β**Glu** |
| *E. coli* glc⁻ | Mauve |
| *E. coli* typiques | Bleu |
| *E. coli* typiques + autres conformes | Bleu foncé |
| Coliformes autres que *E. coli* | Orange |
| *Aeromonas* | Jaune |

### Exemple 12 :

La combinaison des chromogènes est la même que celle de l'exemple 10 mais il a été ajouté, au milieu décrit précedemment, un inhibiteur du pathogène *Aeromonas* pouvant être indifféremment choisi parmi la cefsulodine 0,005 g/l et l'acide nalidixique 0,001 g/l.

| **ACTIVITES ENZYMATIQUES: Glc /** β**Gal /** β**Glu / inhibiteur de *Aeromonas*** | |
|---|---|
| **Souche de microorganismes présente dans l'échantillon liquide** | **Combinaison de chromogènes : Xglc + pNP** β**Gal + Mag** β**Glu** |
| *E. coli* glc⁻ | Jaune |
| *E. coli* typiques | Vert |
| *E. coli* typiques + autres coliformes | Bleu |
| Coliformes autres que *E*. *coli* | Orange |

### Exemple 13 :

La combinaison des chromogènes est la suivante :

| **CHROMOGENES :** | **SUBSTRATS DES ENZYMES :** |
|---|---|
| 5-bromo-4-chloro-3-indoxyl glucuronide (XGlc) + | β-glucuronidase |
| p-nitrophényl β-glucoside (pNPβGlu) | β-glucosidase |

| **ACTIVITES ENZYMATIQUES : Glc /** β**Glu** | |
|---|---|
| **Souche de microorganismes présente dans l'échantillon liquide** | **Combinaison de chromogènes : Xglc + pNP** β**Glu** |
| *E. coli* | Bleu |
| *E. coli* + autres coliformes | Bleu vert |
| Coliformes autres que *E. coli* | Jaune |

### Exemple 14 :

Les enzymes sont les mêmes que dans l'exemple 13 mais un chromophore est différent. La combinaison des chromogènes est la suivante :

| **CHROMOGENES :** | **SUBSTRATS DES ENZYMES :** |
|---|---|
| 5-bromo-4-chloro-3-indoxyl glucuronide (XGlc) + | β-glucuronidase |
| 5-bromo-6-chloro-3-indoxyl β-glucoside (Mag βGlu) | β-glucosidase |

| **ACTIVITES ENZYMATIQUES : Glc /** β**Glu** | |
|---|---|
| **Souche de microorganismes présente dans l'échantillon liquide** | **Combinaison de chromogènes : Xglc + Mag** β**Glu** |
| *E. coli* | Bleu |
| *E. coli* + autres coliformes | Bleu foncé |
| Coliformes autres que *E*. *coli* | Mauve |

### Exemple 15 :

La combinaison des chromogènes est la suivante :

| **CHROMOGENES :** | **SUBSTRATS DES ENZYMES :** |
|---|---|
| 5-bromo-4-chloro-3-indoxyl glucuronide (XGlc) + | β-glucuronidase |
| 5-bromo-6-chloro-3 indoxyl β-galactoside (Mag βGal) | β-galactosidase |
| p-nitrophényl α-glucoside (pNPβGlu) | β-glucosidase |

| **ACTIVITES ENZYMATIQUES: Glc /** β**Gal /** β**Glu** | |
|---|---|
| **Souche de microorganismes présente dans l'échantillon liquide** | **Combinaison de chromogènes : Xglc + Mag** β**Gal + pNP** β**Glu** |
| *E. coli* | Bleu |
| Coliformes autres que *E. coli* | Orange |
| *Enterococcus* | Jaune |

## Revendications

1. Procédé de détection, d'identification et de différenciation d'au moins une souche de microorganismes choisie dans le groupe comprenant les souches : *E. coli glc⁻,* les souches *E*. *coli* typiques, les coliformes autres que *E*. *coli* ou autres que *E. coli* typiques et les bactéries du genre *Aeromonas,* dans un échantillon liquide susceptible de contenir au moins l'une de ces souches, ledit procédé comprenant:
a) le mélange de l'échantillon liquide avec un milieu de sorte d'obtenir un mélange liquide comprenant:
- les nutriments nécessaires à l'incubation de ladite au moins une souche à détecter,
- au moins deux chromogènes, chacun desdits chromogènes étant soit le substrat d'une enzyme exprimée par ladite au moins une souche à détecter soit le substrat d'une enzyme exprimée par une autre souche susceptible de contaminer ledit échantillon et libérant chacun un chromophore sous l'effet de cette enzyme, lesdits chromophores contribuant à la couleur finale dudit mélange liquide,
b) l'incubation du mélange obtenu a l'étape a) pendant 18 à 24 heures à une température de 34°C à 40°C et de préférence à 37°C,
c) la soumission du mélange incubé au rayonnement de la lumière et la lecture de la couleur finale dudit mélange dans les longueurs d'onde du visible, et d) l'identification de ladite au moins une souche de microorganismes en fonction de ladite couleur finale.

2. Le procédé selon la revendication 1, dans lequel ladite enzyme est choisie dans le groupe comprenant la β-D- galactosaminidase, la β-D- glucosaminidase, la β-D-cellobiosidase, la β-D-fucosidase, la α-L- fucosidase, la α -D-galactosidase, la β -D-galactosidase, la β -D- lactosidase, la α-D-maltosidase, la α-D- mannosidase, la α-D-glucosidase, la β-D-glucosidase, la P -D-xylosidase, l'estérase, l'acétate estérase, la butyrate estérase, la carboxylestérase, la caprylate estérase, la choline estérase, la myo-inositol phosphatase, la palmitate estérase, la phosphatase, la diphosphatase, l'aminopeptidase et la sulfatase.

3. Le procédé selon l'une des revendications 1 ou 2, dans lequel ledit chromophore est choisi dans le groupe comprenant O- nitrophényl, P- nitrophényl, Chloro Nitrophényl, Hydroxyphényl, Nitroanilide, Phénolphtaléine et thymophtaléine, Hydroxyquinoline, Cyclohexenoesculétine, Dihydroxyflavone, Catéchol, Résazurin, résofurin, VBzTM, VLM, VLPr, VQM, Indoxyl, 5- bromo-4-chloro-3-indoxyl, 5-bromo-6-chloro-3-indoxyl, 6-chloro-3 -indoxyl, 6-fluoro-3-indoxyl, 5-Iodo-3-indoxyl, N-Méthylindoxyl.

4. Le procédé selon l'une quelconque des revendications 1 à 3, dans lequel l'un des au moins deux chromogènes est le 5- bromo-4-chloro-3-indoxyl glucuronide.

5. Le procédé selon la revendication 4, **caractérisé en ce que** ledit procédé permet la détection, l'identification et la différentiation d'au moins une souche de microorganisme dans un mélange susceptible de comprendre une souche de microorganisme choisie dans le groupe constitué de *E*. *coli glc⁻ ,* les souches *E*. *coli* typiques, les coliformes autres que *E*. *coli* et les bactéries du genre *Aeromonas,* et **en ce que** ledit milieu comprend, à titre d'agents chromogènes :
a) le 5- bromo-4-chloro-3 -indoxyl glucuronide, le p-nitrophényl-α-galactoside et le 5-bromo-6-chloro-3-indoxyl β-glucoside;
b) le 5- bromo-4-chloro-3-indoxyl glucuronide, le 5- bromo-6-chloro-3-indoxyl -α-galactoside et le p-nitrophényl-β-glucoside ;
c) le 5- bromo-4-chloro-3-indoxyl glucuronide, le 5- bromo-6-chloro-3-indoxyl -β-glucoside et le p-nitrophényl-p- galactoside ; ou
d) le 5- bromo-4-chloro-3 -indoxyl glucuronide, le 5-bromo-6-chloro-3-indoxyl-β-galactoside et le p-nitrophényl-β-glucoside.

6. Le procédé selon la revendication 4, **caractérisé en ce que** ledit procédé permet la détection, l'identification et la différentiation d'au moins une souche de microorganisme dans un mélange susceptible de comprendre une souche de microorganisme choisie dans le groupe constitué de *E*. *coli,* les coliformes autres que *E*. *coli* et les bactéries du genre *Aeromonas,* et **en ce que** ledit milieu comprend, à titre d'agents chromogènes :
a) le 5- bromo-4-chloro-3 -indoxyl glucuronide, le p-nitrophényl-α-galactoside et le 5-bromo-6-chloro-3-indoxyl β-galactoside ; ou
b) ) le 5- bromo-4-chloro-3-indoxyl glucuronide, le 5- bromo-6-chloro-3-indoxyl α-galactoside et le p-nitrophényl- β galactoside.

7. Le procédé selon la revendication 4, **caractérisé en ce que** ledit procédé permet la détection, l'identification et la différentiation d'au moins une souche de microorganisme dans un mélange susceptible de comprendre une souche de microorganisme choisie dans le groupe constitué de *E. coli* et les coliformes autres que *E. coli*, et **en ce que** ledit milieu comprend, à titre d'agents chromogènes:
a) le 5-bromo-4-chloro-3-indoxyl glucuronide et le p-nitrophényl β-galactoside
b) ) le 5-bromo-4-chloro-3-indoxyl glucuronide et le 5-bromo-6-chloro-3-indoxyl β-galactoside ;
c) le 5-bromo-4-chloro-3-indoxyl glucuronide et le p-nitrophényl β-glucoside ; ou
d) le 5- bromo-4-chloro-3-indoxyl glucuronide et le 5-bromo-6-chloro-3-indoxyl β-glucoside.

8. Le procédé selon la revendication 7, **caractérisé en ce que** ledit milieu comprend, à titre d'agents chromogènes le 5-bromo-4-chloro-3-indoxyl glucuronide et le p-nitrophényl β-galactoside.

9. Procédé selon l'une quelconque des revendications 1 à 8, dans lequel l'échantillon liquide est de l'eau de préférence de l'eau potable.

## Claims

1. A process for the detection, identification and differentiation of at least one of the microorganism strain chosen in the group comprising : the strains *E*. *coli* glc-, *typical E. coli* strains, coliforms other than *E*. *coli* or other than typical *E. coli,* and the *Aeromonas* genus bacteria, in a liquid sample likely to contain at least one of these strains, said process comprising :
a) the mixture of the liquid sample with a medium so as to obtain a liquid mixture comprising :
- the nutrients required for the incubation of said at least one strain to be detected,
- at least two chromogens, each of said chromogens being either the substrate of an enzyme expressed by the at least one strain to be detected either the substrate of an enzyme expressed by another strain likely to contaminate said sample and each releasing a chromophore under the effect of this enzyme,
said chromophores contributing to the final color of said liquid mixture,
b) incubating the mixture obtained at step a) during 18 to 24 hours at a temperature of 34°C to 40°C and preferably at 37°C,
c) the submission of the incubated mixture to the light radiation and reading the final color of said mixture at visible wavelengths, and
d) the identification of said at least one microorganism strain according to said final color.

2. A process according to claim 1, wherein said enzyme is chosen among the group comprising β-D-galactosaminidase, β-D-glucosaminidase, β-D-cellobiosidase, β-D-fucosidase, α-L-fucosidase, α-D-galactosidase, β-D-galactosidase, β-D-lactosidase, α-D-maltosidase, α-D-mannosidase, α-D-glucosidase, β-D-glucosidase, β-D-xylosidase, esterase, acetate esterase, butyrate esterase, carboxyl esterase, caprylate esterase, choline esterase, myo-inositol phosphatase, palmitate esterase, phosphatase, diphosphatase, aminopeptidase and sulfatase.

3. A process according to one of the claims 1 or 2, wherein said chromophore is chosen among the group comprising O-nitrophenyl, P-nitrophenyl, chloro-nitrophenyl, hydroxyphenyl, nitroanilide, phenolphthalein and thymophthalein, hydroxyquinoline, cyclohexane-esculetin, dihydroxyflavone, catechol, resazurin, resofurin, VBzTM, VLM, VLPr, VQM, indoxyl, 5-bromo-4-chloro-3-indoxyl, 5-bromo-6-chloro-3-indoxyl, 6-chloro-3-indoxyl, 6-fluoro-3-indoxyl, 5-Iodo-3-indoxyl and N-methylindoxyl.

4. A process according to one of any claims 1 to 3, wherein one of the at least two chromogenes is 5-bromo 4-chloro 3-indoxyl glucuronide.

5. A process according to claim 4, **characterised in that** said process allows the detection, the identification and the differentiation of at least one microorganism strain in a mixture likely to comprise a microorganism strain chosen in the group constituted of *E.coli glc⁻,* typical *E. coli* strains, coliforms other than *E. coli* and bacteria of *Aeromonas* genus, and wherein said medium comprises, as chromogenic agents :
a) 5-bromo-4-chloro-3-indoxyl glucuronide, p-nitrophenyl-α-galactoside and 5-bromo-6-chloro-3-indoxyl β-glucoside ;
b) 5-bromo-4-chloro-3-indoxyl glucuronide, 5-bromo-6-chloro-3-indoxyl-α-galactoside and p-nitrophenyl-β-glucoside;
c) 5-bromo-4-chloro-3-indoxyl glucuronide, 5-bromo-6-chloro-3-indoxyl-β-glucoside and p-nitrophenyl-β-galactoside ; or
d) 5-bromo-4-chloro-3-indoxyl glucuronide, 5-bromo-6-chloro-3-indoxyl- β-galactoside and p-nitrophenyl-β-glucoside.

6. Process according to claim 4, **characterised in that** said process allows the detection, the identification and the differentiation of at least one microorganism strain in a mixture likely to comprise a microorganism strain chosen in the group constituted of *E. coli,* coliforms other than *E. coli* and bacteria of *Aeromonas* genus, and wherein said medium comprises, as chromogenic agents :
a) 5-bromo-4-chloro-3-indoxyl glucuronide, p-nitrophenyl-α-galactoside and 5-bromo-6-chloro-3-indoxyl β-galactoside ; or
b) 5-bromo-4-chloro-3-indoxyl glucuronide, 5-bromo-6-chloro-3-indoxyl-α-galactoside and p-nitrophenyl-β-galactoside.

7. Process according to claim 4, **characterised in that** said process allows the detection, the identification and the differentiation of at least one microorganism strain in a mixture likely to comprise a microorganism strain chosen in the group constituted of *E. coli* and coliforms other than *E. coli* and wherein said medium comprises as chromogenic agents :
a) 5-bromo-4-chloro-3-indoxyl glucuronide and p-nitrophényl β-galactoside ;
b) 5-bromo-4-chloro-3-indoxyl glucuronide and 5-bromo-6-chloro-3-indoxyl β-galactoside ;
c) 5-bromo-4-chloro-3-indoxyl glucuronide and p-nitrophenyl β-glucoside ; or
d) 5-bromo-4-chloro-3-indoxyl glucuronide and 5-bromo-6-chloro-3-indoxyl β-glucoside.

8. Process according to claim 7, **characterised in that** said medium comprises, as chromogenic agents 5-bromo-4-chloro-3-indoxyl glucuronide and p-nitrophenyl β-galactoside.

9. Process according to any one of claims 1 to 8, wherein the liquid sample is water, preferably drinking water.

## Patentansprüche

1. Verfahren zur Detektion, Identifizierung und Differenzierung mindestens eines Stammes von Mikroorganismen, welcher ausgewählt ist aus der Gruppe umfassend die Stämme: *E*. *coli glc⁻,* die typischen *E*. *coli-*Stämme, die Coliformen außer *E. coli* oder außer typischen *E. coli* und die Bakterien der Gattung *Aeromonas,* in einer flüssigen Probe, welche möglicherweise mindestens einen dieser Stämme enthält, wobei das Verfahren umfasst:
a) das Mischen der flüssigen Probe mit einem Medium derart, dass eine flüssige Mischung erhalten wird, welche umfasst:
- die Nährstoffe, die für die Inkubation des mindestens einen zu detektieren Stamms erforderlich sind,
- mindestens zwei Chromogene, wobei jedes dieser Chromogene entweder das Substrat eines Enzyms ist, das durch den mindestens einen zu detektieren Stamm exprimiert wird, oder das Substrat eines Enzyms ist, das durch einen anderen Stamm, der die Probe möglicherweise infiziert, exprimiert wird, und unter der Wirkung dieses Enzyms jeweils einen Chromophor freisetzt, wobei die Chromophore zu der endgültigen Farbe der flüssigen Mischung beitragen,
b) das Inkubieren der in Schritt a) erhaltenen Mischung während 18 bis 24 Stunde bei einer Temperatur von 34°C bis 40°C und vorzugsweise bei 37°C,
c) das Einwirkenlassen der inkubierten Mischung mit Lichtstrahlung und das Ablesen der endgültigen Farbe der Mischung in den Wellenlängen des sichtbaren Lichts und
d) das Identifizieren des mindestens einen Stammes von Mikroorganismen abhängig von der endgültigen Farbe.

2. Verfahren nach Anspruch 1, in welchem das Enzym aus der Gruppe umfassend β-D-Galactosaminidase, β-D-Glucosaminidase, β-D-Cellobiosidase, β-D-Fucosidase, α-L-Fucosidase, α-D-Galactosidase, β-D-Galactosidase, β-D-Lactosidase, α-D-Maltosidase, α-D-Mannosidase, α-D-Glucosidase, β-D-Glucosidase, β-D-Xylosidase, Esterase, Acetatesterase, Butyratesterase, Carboxylesterase, Caprylatesterase, Cholinesterase, Myoinositolphosphatase, Palmitatesterase, Phosphatase, Diphosphatase, Aminopeptidase und Sulfatase ausgewählt ist.

3. Verfahren nach einem der Ansprüche 1 oder 2, in welchem der Chromophor ist aus der Gruppe umfassend o-Nitrophenyl, p-Nitrophenyl, Chlornitrophenyl, Hydroxyphenyl, Nitroanilid, Phenolphthtalein und Thymophthalein, Hydroxychinolin, Cyclohexenoesculetin, Dihydroxyflavon, Catechol, Resazurin, Resofurin, VBzTM, VLM, VLPr, VQM, Indoxyl, 5-Brom-4-chlor-3-indoxyl, 5-Brom-6-chlor-3-indoxyl, 6-Chlor-3-indoxyl, 6-Fluor-3-indoxyl, 5-lod-3-indoxyl, N-Methylindoxyl ausgewählt ist.

4. Verfahren nach einem der Ansprüche 1 bis 3, in welchem eines der mindestens zwei Chromogene 5-Brom-4-chlor-3-indoxylglucuronid ist.

5. Verfahren nach Anspruch 4, **dadurch gekennzeichnet, dass** das Verfahren die Detektion, die Identifizierung und die Differenzierung mindestens eines Mikroorganismenstammes in einer Mischung ermöglicht, welche möglicherweise einen Mikroorganismenstamm umfasst, welcher aus der Gruppe bestehend aus *E. coli glc⁻,* den typischen *E. coli*-Stämmen, den Coliformen außer *E. coli* und den Bakterien der Gattung *Aeromonas* ausgewählt ist, und dass das Medium als chromogene Mittel:
a) 5-Brom-4-chlor-3-indoxylglucuronid, p-Nitrophenyl-αgalactosid und 5-Brom-6-chlor-3-indoxyl-β-glucosid;
b) 5-Brom-4-chlor-3-indoxylglucuronid, 5-Brom-6-chlor-3-indoxyl-α-galactosid und p-Nitrophenyl-β-glucosid;
c) 5-Brom-4-chlor-3-indoxylglucuronid, 5-Brom-6-chlor-3-indoxyl-β-glucosid und p-Nitrophenyl-β-galactosid; oder
d) 5-Brom-4-chlor-3-indoxylglucuronid, 5-Brom-6-chlor-3-indoxyl-β-galactosid und p-Nitrophenyl-β-glucosid umfasst.

6. Verfahren nach Anspruch 4, **dadurch gekennzeichnet, dass** das Verfahren die Detektion, die Identifizierung und die Differenzierung mindestens eines Mikroorganismenstammes in einer Mischung ermöglicht, welche möglicherweise einen Mikroorganismenstamm umfasst, welcher aus der Gruppe bestehend aus *E. coli,* den Coliformen außer *E. coli* und den Bakterien der Gattung *Aeromonas* ausgewählt ist, und dass das Medium als chromogene Mittel:
a) 5-Brom-4-chlor-3-indoxylglucuronid, p-Nitrophenyl-αgalactosid und 5-Brom-6-chlor-3-indoxyl-β-galactosid; oder
b) 5-Brom-4-chlor-3-indoxylglucuronid, 5-Brom-6-chlor-3-indoxyl-α-galactosid und p-Nitrophenyl-β-galactosid umfasst.

7. Verfahren nach Anspruch 4, **dadurch gekennzeichnet, dass** das Verfahren die Detektion, die Identifizierung und die Differenzierung mindestens eines Mikroorganismenstammes in einer Mischung ermöglicht, welche möglicherweise einen Mikroorganismenstamm umfasst, welcher aus der Gruppe bestehend aus *E. coli* und den Coliformen außer *E. coli* ausgewählt wird, und dass das Medium als chromogene Mittel:
a) 5-Brom-4-chlor-3-indoxylglucuronid und p-Nitrophenyl-β-galactosid;
b) 5-Brom-4-chlor-3-indoxylglucuronid und 5-Brom-6-chlor-3-indoxyl-β-galactosid;
c) 5-Brom-4-chlor-3-indoxylglucuronid und p-Nitrophenyl-β-glucosid; oder
d) 5-Brom-4-chlor-3-indoxylglucuronid und 5-Brom-6-chlor-3-indoxyl-β-glucosid
umfasst.

8. Verfahren nach Anspruch 7, **dadurch gekennzeichnet, dass** das Medium als chromogene Mittel 5-Brom-4-chlor-3-indoxylglucuronid und p-Nitrophenyl-β-galactosid umfasst.

9. Verfahren nach einem der Ansprüche 1 bis 8, in welchem die flüssige Probe Wasser, vorzugsweise Trinkwasser, ist.
